# EUROPÄISCHE PATENTSCHRIFT

(11) **EP 0 514 416 B1**
(45) Veröffentlichungstag und Bekanntmachung des Hinweises auf die Patenterteilung: **29.06.1994**
(21) Anmeldenummer: 91903303.5
(22) Anmeldetag: 05.02.1991
(51) Int. Cl.: C07K 15/04, C07K 1/14, A61K 37/02

(54) **BAKTERIELLE PEPTIDFRAKTION MIT IMMUNSTIMULIERENDER WIRKUNG, VERFAHREN ZUR HERSTELLUNG DERSELBEN UND IHRE VERWENDUNG**
BACTERIAL PEPTIDE FRACTION WITH AN IMMUNOSTIMULATORY EFFECT, PROCESS FOR MAKING IT AND ITS USE
FRACTION DE PEPTIDE BACTERIEN A EFFET IMMUNOSTIMULANT, SON PROCEDE DE PRODUCTION ET SON UTILISATION

(30) Priorität: 08.02.1990 DE 4003815
(43) Veröffentlichungstag der Anmeldung: 25.11.1992
(73) Patentinhaber: Pulverer, Gerhard, Prof. Dr.Dr.h.c., 50858 Köln (DE)
(72) Erfinder: PULVERER, Gerhard, D-5000 Köln 40 (DE); ROSZKOWSKI, Waldemar, Warsaw (PL)
(74) Vertreter: Werner, Hans-Karsten, Dr.Dipl.-Chem.
(86) Internationale Anmeldenummer: EP9100217
(87) Internationale Veröffentlichungsnummer: WO9112274

(56) Entgegenhaltungen:
- INFLAMMATION, vol. 4, no. 3, September 1980, Plenum Press, New York, NY (US); G.F. WEBSTER et al., pp. 261-269#
- ZBL. BAKT. HYG., vol. A 270, 1988; G. PULVERER et al., pp. 246-251#
- ZBL. BAKT., vol. 272, no. 3, March 1990, Gustav Fischer Verlag; G. PULVERER et al., pp. 318-327#
- ZBL. BAKT. HYG., vol. A 270, 1988; K. ROSZKOWSKI et al., pp. 270-279#

## Beschreibung

Gegenstand der vorliegenden Erfindung ist eine bakterielle Peptidfraktion mit immunstimulierender Wirkung erhältlich aus kommensalischen bzw. symbiotischen Mikroorganismen, die der physiologischen Mikroflora des Verdauungstraktes, der Haut oder des Atmungstraktes angehören, sowie Verfahren zu ihrer Herstellung und ihre Verwendung zur Herstellung von Arzneimitteln.

Es war bereits früher beobachtet worden, daß nach Elimination der aeroben und anaeroben physiologischen Mikroflora des Gastrointestinaltraktes, eine Einschränkung der Peritonealmakrophagen-Aktivität (Phagozytose, Chemotaxis, Bakterizidie, zytostatischer Effekt) sowie eine Reduktion der Lymphozytenproliferation (mesenteriale Lymphknoten, Thymus, Milz) eintritt; vgl. ZBL. BAKT. HYG. A 270, 270 bis 279 (1988). Es wurde jetzt überraschenderweise festgestellt, daß dieser Einfluß der Mikroorganismen des Verdauungstraktes auf das Immunsystem darauf zurückzuführen ist, daß diese Mikroorganismen in erheblichen Mengen eine Peptidfraktion mit immunstimulierender Wirkung produzieren und abscheiden. Diese Peptidfraktion ist wasserlöslich und hat ein Molekulargewicht von < 10.000 D. Diese Peptidfraktion läßt sich von der Biomasse abtrennen und beispielsweise auf einer Sephadex® G-25 Säule mit Wasser als Elutionsmittel auftrennen. Es wurde weiterhin festgestellt, daß eine derartige Peptidfraktion nicht nur von den Mikroorganismen des Verdauungstraktes, sondern generell von kommensalischen bzw. symbiotischen Mikroorganismen des Verdauungstraktes, der Haut oder des Atmungstraktes produziert und an den Wirt abgegeben wird. Diese Aussage gilt für Kommensalen und Symbionten sowohl von Tieren als auch des Menschen.

Es wurde weiterhin festgestellt, daß diese Peptidfraktion in der Lage ist, immunstimulierend zu wirken. Die Peptidfraktion kann deshalb verwendet werden insbesondere zur Aktivierung der Lymphozyten, zur Reifung und Differenzierung von Lymphozyten und Thymozyten, Stimulierung von Makrophagen und Monozyten, der Behandlung von klinischen Symptomen wegen zu geringer Zahlen von T-Helferzellen, der Gegenregulierung von negativen Immuneffekten durch Verdauungsstörungen oder durch Entleerung des Darmtraktes vor Operationen oder als Folge einer Breitbandantibiotika-Therapie.

In den meisten Fällen liegt das Molekulargewicht dieser Peptidfraktion sogar bei < 6.500 D.

Besonders eingehende Untersuchungen liegen vor mit der Peptidfraktion aus Propionibakterium acnes und Staphylococcus saphrophyticus. Vorläufige Untersuchungen mit anderen derartigen Mikroorganismen (z.B. Bacteroides specialis, Lactobacillus specialis, Clostridium specialis) haben gezeigt, daß auch diese in der Lage sind, eine Peptidfraktion mit immunstimulierender Wirkung herzustellen und abzuscheiden.

Analysen der lymphatischen Zellen von Thymus und Milz zeigen nach einer Injektion mit dieser Peptidfraktion einen signifikanten Anstieg der T-Helferlymphozyten. Die Immunzellfunktion und Proliferationsfähigkeit lymphatischer Zellen im Thymus und der Milz werden durch diese Peptidfraktion wiederhergestellt. Cortison bewirkt bekanntlich eine signifikante Reduktion von Thymozyten. Eine Injektion dieser Peptidfraktion führt zu einem Wiederanstieg der Thymozytenzahl und egalisiert damit den immunsuppressiven Cortison-Effekt.

Die Herstellung dieser Peptidfraktion ist einfach und leicht reproduzierbar. Zunächst werden die verwendeten Mikroorganismen auf üblichen Nährböden gezüchtet und danach vom Nährboden durch Zentrifugieren abgetrennt. Die gesammelten Überstände können lyophylisiert, in Wasser wieder aufgelöst und dialysiert werden, so daß nur die Fraktionen mit einem Molekulargewicht von < 10.000 D, vorzugsweise < 6.500 D, übrig bleiben. Diese Fraktionen können am einfachsten aus wäßriger Lösung auf einer Sephadex® G-25 Säule aufgebracht und mit Wasser eluiert werden. Die Fraktionen mit der höchsten Extinktion bei 280 nm werden abgetrennt und gewünschtenfalls weiter aufgearbeitet. Diese Fraktionen können aber bereits in dieser Form eingefroren und bei Bedarf wieder aufgetaut werden. Diese Peptidfraktion kann injiziert, jedoch auch oral appliziert werden, sofern dafür gesorgt wird, daß sie unversehrt bis zur Darmwand gelangt.

Bei der Applikation werden nicht nur die oben aufgezählten Wirkungen erzielt, sondern darüber hinaus beobachtet, daß im Tierversuch das Gewicht der Thymusdrüse ansteigt.

## Patentansprüche

1. Bakterielle Peptidfraktion mit stimulierender Wirkung auf das lymphatische Immunsystem erhältlich aus kommensalischen bzw. symbiotischen Mikroorganismen, die der physiologischen Mikroflora des Verdauungstraktes, der Haut oder des Atmungstraktes von Mensch und Tier zugehören, durch Züchtung auf üblichen Nährböden, Abtrennung der wasserlöslichen Peptidfraktionen mit dem Molekulargewicht von < 10.000 D von der Biomasse und Auftrennung auf einer Sephadex® G-25 _{S}äule mit Wasser als Elutionsmittel, wobei die Fraktionen mit der höchsten Extinktion bei 280 nm gewonnen werden.

2. Bakterielle Peptidfraktion gemäß Anspruch 1 aus Propionibakterium acnes oder Staphylococcus saphrophyticus.

3. Bakterielle Peptidfraktion gemäß Anspruch 1 oder 2 mit einem Molekulargewicht < 6.500 D.

4. Verfahren zur Herstellung einer bakteriellen Peptidfraktion mit stimulierender Wirkung auf das lymphatische Immunsystem, dadurch gekennzeichnet, daß kommensalische bzw. symbiotische Mikroorganismen, die der physiologischen Mikroflora des Verdauungstraktes, der Haut oder des Atmungstraktes von Mensch und Tier angehören, auf üblichen Nährböden gezüchtet, die wasserlöslichen Peptidfraktionen mit einem Molekulargewicht < 10.000 D von der Biomasse abgetrennt, auf einer Sephadex® G-25 Säule mit Wasser als Elutionsmittel aufgetrennt und die Fraktionen mit der höchsten Extinktion bei 280 nm gewonnen werden.

5. Verwendung der bakteriellen Peptidfraktion gemäß der Ansprüche 1, 2, 3 oder 4 zur Herstellung von Arzneimitteln mit immunstimulierender Wirkung, insbesondere zur Aktivierung der Lymphozyten, zur Reifung und Differenzierung von Lymphozyten und Thymozyten, Stimulierung von Makrophagen und Monozyten, der Behandlung von klinischen Symptomen wegen zu geringer Zahlen von T-Helferzellen, der Gegenregulierung von negativen Immuneffekten durch Verdauungsstörungen oder durch Entleerung des Darmtraktes vor Operationen oder als Folge einer Breitbandantibiotika-Therapie.

## Claims

1. A bacterial peptide fraction having stimulating activity on the lymphatic immune system, obtainable from commensal or symbiotic microorganisms, which are part of the physiological microflora of the digestive tract, the skin or the respiratory tract in humans and animals, by growth on common culture media, removal of water-soluble peptide frcations having a molecular weight of < 10,000 D from the biomass and separation on a Sephadex® G-25 column using water as the eluent, the fractions having the highest extinction at 280 nm being recovered.

2. The bacterial peptide fraction according to claim 1 from Propionibacterium acnes or Staphylococcus saphrophyticus.

3. The bacterial peptide fraction according to claim 1 or 2 having a molecular weight of < 6,500 D.

4. A process for preparing a bacterial peptide fraction having stimulating activity on the lymphatic immune system, characterized in that commensal or symbiotic microorganisms, which are part of the physiological microflora of the digestive tract, the skin or the respiratory tract in humans and animals, are grown on common culture media, water-soluble peptide fractions having a molecular weight of < 10,000 D are removed from the biomass, separated on a Sephadex® G-25 column using water as the eluent, and the fractions having the highest extinction at 280 nm are recovered.

5. Use of the bacterial peptide fraction according to claims 1, 2, 3, or 4 for preparing drugs having immunostimulating activity, in particular, for activating lymphocytes, for maturing and differentiating lymphocytes and thymocytes, for stimulating macrophages and monocytes, for treating clinical symptoms due to low number of helper T-cells, for counter-regulating negative immunoeffects caused by digestive disorders or by evacuation of the intestinal tract prior to operation or as a consequence of broad spectrum antibiotics therapy.

## Revendications

1. Fraction de peptide bactérien ayant un effet stimulant sur le système immunitaire lymphatique, qu'on peut obtenir à partir de micro-organismes commensaux ou de micro-organismes symbiotiques qui appartiennent à la microflore physiologique du tube digestif, de la peau ou de l'appareil respiratoire de l'homme ou de l'animal, par mise en culture sur des milieux nutritifs usuels, isolement des fractions peptidiques solubles dans l'eau et ayant un poids moléculaire < 10 000 D de la biomasse, et séparation de celles-ci sur une colonne Sephadex^{(R)} G-25 en utilisant de l'eau comme éluant, les fractions ayant l'extinction la plus élevée à 280 nm étant obtenues.

2. Fraction de peptide bactérien selon la revendication 1, obtenue à partir de *Propionibacterium acnes* ou de *Staphylococcus saphrophyticus.*

3. Fraction de peptide bactérien selon la revendication 1 ou 2, ayant un poids moléculaire < 6 500 D.

4. Procédé de préparation d'une fraction de peptide bactérien ayant un effet stimulant sur le système immunitaire lymphatique, caractérisé en ce qu'on cultive sur des milieux nutritifs usuels des micro-organismes commensaux ou des micro-organismes symbiotiques qui appartiennent à la microflore physiologique du tube digestif, de la peau ou de l'appareil respiratoire de l'homme ou de l'animal, on isole les fractions peptidiques solubles dans l'eau et ayant un poids moléculaire < 10 000 D de la biomasse, on les sépare sur une colonne Sephadex^{(R)} G-25 en utilisant de l'eau comme éluant, et on obtient les fractions ayant l'extinction la plus élevée à 280 nm.

5. Utilisation de la fraction de peptide bactérien selon la revendication 1, 2, 3 ou 4 pour la préparation de médicaments ayant un effet immunostimulant, en particulier pour l'activation des lymphocytes, la maturation et la différenciation des lymphocytes et des thymocytes, la stimulation des macrophages et des monocytes, le traitement de symptômes cliniques dus à des nombres trop petits de cellules T auxiliaires, la contre-régulation d'effets immunitaires négatifs dus à des troubles digestifs ou au fait de vider le tube digestif avant une opération, ou comme conséquence d'une thérapie avec des antibiotiques à spectre large.
